Europäisches Patentamt

European Patent Office

Office européen des brevets

(11) Numéro de publication: **0 201 413**
**A1**

## (12) DEMANDE DE BREVET EUROPEEN

(21) Numéro de dépôt: **86400940.2**

(22) Date de dépôt: **29.04.86**

(51) Int. Cl.4: **A61K 39/385**

(30) Priorité: **02.05.85 FR 8506679**

(43) Date de publication de la demande:
**17.12.86 Bulletin 86/46**

(84) Etats contractants désignés:
**AT BE CH DE FR GB IT LI LU NL SE**

(71) Demandeur: **INSTITUT PASTEUR Fondation reconnue d'utilité publique**
**28 rue du Docteur Roux**
**F-75724 Paris Cédex 15(FR)**
Demandeur: **CENTRE NATIONAL DE LA RECHERCHE SCIENTIFIQUE (CNRS)**
**15, Quai Anatole France**
**F-75007 Paris(FR)**

(72) Inventeur: **Avrameas, Eustrate Stratis**
**1 rue Sarasate**
**F-75015 Paris(FR)**
Inventeur: **Guilbert, Brigitte**
**3 rue de Berry**
**F-94150 Chevilly-Larue(FR)**
Inventeur: **Mahana, Wahib**
**14 rue des Hautes Formes**
**F-75013 Paris(FR)**

(74) Mandataire: **Phélip, Bruno et al**
**c/o Cabinet Harlé & Phélip 21, rue de la Rochefoucauld**
**F-75009 Paris(FR)**

(54) **Composés suscitant la formation d'anticorps, compositions immunogéniques et compositions immunoprotectrices les contenant et procédé d'obtention d'anticorps les mettant en oeuvre.**

(57) Les composés résultent de la fixation d'un agent antigénique sur une molécule-support, qui administrée seule à l'individu adulte ne déclencherait pas de réaction immunitaire.

De telles molécules-support proviennent d'un individu de la même espèce que celui à qui on administrera le composé selon l'invention pour induire la formation d'anticorps chez cet individu. Au moins une administration devra avoir lieu dans la période néo-natale. La molécule-support préférée est une protéine et spécialement la sérum albumine.

Application comme agent de vaccination ou pour obtenir des anticorps de spécificité donnée chez l'animal in vivo ou ex-vivo à des fins de préparation de réactifs pour des dosages immunologiques.

La présente invention concerne des composés dont l'administration à un individu suscite la formation d'anticorps dirigés contre l'antigène ou l'haptène dont ils dérivent, ainsi que les compositions qui contiennent de tels composés, qui sont utilisables comme immunogènes et immunoprotecteurs.

On considérait jusqu'à présent qu'un haptène devait être couplé à une molécule-support hétérologue pour que son administration à un organisme humain ou animal déclenche la formation d'anticorps dirigés contre cet haptène; le couplage à une molécule provenant d'un individu de même espèce était considéré comme ne pouvant pas induire la formation d'anticorps dirigés contre l'haptène.

On a aussi constaté que des substances antigéniques en elles-mêmes, que ce soit des antigènes figurés tels que virus, champignons, bacilles et autres bactéries ou des antigènes solubles tels que les anatoxines diphtériques ou tétaniques, des polysaccharides ou des protéines hétérologues, ont souvent des effets secondaires non négligeables si on doit les administrer seules en une quantité suffisante pour déclencher une réaction immunitaire d'utilité pratique. Ainsi dans le cas des vaccinations non seulement les injections sont repétées, mais encore des immunostimulants sont souvent administrés simultanément au réactif antigènique déclenchant, pour diminuer la dose vaccinante et les réactions secondaires; on peut citer parmi les immunostimulants les dérivés de l'alumine, le glycérol, ou le muramyldipeptide et ses analogues actuellement à l'étude. Ou encore, dans le cas où l'on veut, chez l'animal, stimuler la formation des lymphocytes B secréteurs d'anticorps dirigés contre une substance antigénique particulière, par exemple pour préparer et cloner des hybridomes, on administre simultanément à la substance, de l'adjuvant de Freund complet ou incomplet.

Par ailleurs, on sait que la plupart des vaccins actuels, à base d'antigènes figurés détoxifiés ou d'antigènes synthétiques conjugués, lorsqu'ils sont administrés à des nouveaux-nés ne protègent pas ceux-ci contre les infections ultérieures; chez l'homme, seul la vaccination par le BCG est pratiquée efficacement dans les premiers mois de la vie.

La présente invention a donc un caractère inattendu. Elle a en effet pout objet des composés qui déclenchent la formation d'anticorps dirigés contre leur élément antigénique lorsqu'ils sont administrés au nouveau-né, et qui injectés par la suite au même individu devenu adulte, donneront une réponse immunitaire supérieure, en intensité et spécificité, à celle qui pourrait apparaître lors de l'administration d'un antigène de type connu.

Les composés selon l'invention, utiles pour induire la sécrétion d'anticorps chez un individu, sont constituées d'un agent antigénique fixé ou lié à une molécule support d'un type particulier, c'est-à-dire un molécule ne pouvant déclencher une réaction immunitaire lorsqu'elle est administrée seule à l'individu adulte à traiter.

Un autre objet de l'invention est constitué des compositions dans lesquelles les composés sont associés à un véhicule inerte convenant à leur administration à l'organisme où on désire stimuler la formation d'anticorps dirigés contre l'agent antigénique, que se soit pour traiter ou protéger ledit organisme, pour isoler des anticorps ou les cellules productrices de ces anticorps.

Dans la mesure où la première administration doit être effectuée chez le nouveau-né, la dose unitaire sera adaptée.

Par agent antigénique, on entend ici d'une part les composés susceptibles de déclencher à eux seuls la formation d'anticorps, c'est-à-dire les antigènes figurés, tels bactéries, virus, champignons et autres microorganismes, les antigènes solubles, tels anatoxines, allergènes, protéines hétérologues, polysaccharides, glycolipides et glycoprotéines et d'autre part les haptènes, c'est-à-dire des déterminants antigéniques (ou épitopes)-,peptides, hormones et autres petites molécules naturelles et synthétiques,tels que les médicaments.

Un tel agent antigénique peut être couplé chimiquement à la molécule-support, soit directement lorsqu'il comporte des fonctions réactives, soit par l'intermédiaire d'un agent connu de couplage des macromolécules,tel que le glutaraldéhyde,la benzoquinone,les carbodiimides,le periodate,les dimaleimides et dérivés;le couplage est réalisé d'une manière connue en soi, de telle sorte que les épitopes de l'agent antigénique ne soient pas modifiés. On peut fixer un ou plusieurs agents antigéniques,de même type ou de type différents,sur la molécule-support. La fixation de l'agent antigénique sur la molécule-support peut aussi être effectuée par des liaisons non-covalentes.

La molécule-support est une molécule qui administrée dans l'organisme de l'individu adulte considéré ne déclencherait pas de réaction immunitaire; c'est donc une molécule qui doit provenir de l'organisme considéré lui-même ou d'un individu de la même espèce animale,lorsque ladite molécule a des caractéristiques antigéniques conservées dans l'espèce.Comme support,on peut utiliser des macromolécules autologues mais on préfère les protéines,et plus spécialement l'albumine du sérum,abondante et facile à obtenir.

Selon l'invention, on préfére que le degré de substitution du support ,en particulier de la protéine, par l'agent antigénique,par exemple un haptène, soit le plus élevé possible . Ceci a été démontré, notamment,dans le cas du couplage du groupement TNP sur la sérum albumine de souris - (SSA)ou sur la sérum albumine bovine (BSA)- ,comme on le verra ci-après.

Les composés,selon l'invention,associés à un véhicule convenable,sont administrés à l'individu nouveau-né chez lequel on veut déclencher la formation d'anticorps dirigés contre l'agent antigénique qui en est un élément. La réponse immunitaire est nette et les anticorps formés particulièrement spécifiques.

On peut utiliser ces composés dans des compositions pharmaceutiques pour protéger beaucoup plus précocement un nouveau-né contre des infections diverses et tout particulièrement contre des maladies infantiles comme les infections à méningocoques B, ou hémophilus B.

Une restimulation du même individu est possible ultérieurement, même à l'âge adulte, par de nouvelles injections de la composition et ceci est particulièrement surprenant, alors qu'on sait qu'un individu n'ayant reçu aucune injection d'un composé conforme à l'invention dans la période néonatale ne produit pas d'anticorps contre l'agent antigénique, élément du composé, si ce dernier lui est administré à l'âge adulte.

L'utilisation des composés selon l'invention pour stimuler les défenses immunitaires contre un agent infectieux ou vacciner un individu est d'autant plus avantageuse que la molécule-support n'induit aucune réaction immunitaire nocive dirigée contre elle, ce qui diminue sensiblement les réactions secondaires de l'individu.

On peut utiliser les composés de l'invention comme réactif d'immunisation d'animaux de laboratoire pour préparer de façon connue en soi de grandes quantités d'anticorps soit directement à partir du sérum des animaux traités avec ce réactif soit par culture des hybridomes obtenus à partir des lymphocytes B prélevés sur des animaux traités. De tels anticorps pourraient servir, notamment comme réactifs dans des dosages immunoenzymatiques ou radioimmunologiques dont le principe est bien connu.

Dans ce qui suit, on décrit des exemples de réalisation de l'invention.

L'haptène choisi à titre d'exemple est le groupe-trinitrophényl ou TNP, classiquement utilisé en recherche immunologique, et considéré comme un excellent modèle d'haptène, tout comme la fluorescéine ou l'acide p-aminophénylarsonique. On peut à ce sujet se référer à l'ouvrage "Methods in immunology and immuno-chemistry" Vol. 1 et 2 - (1967) Acad. Press, de Curtis A. Williams et Merril W. Chase. L'haptène a été couplé à la protéine-support sans agent de couplage intermédiaire, étant donné la présence de fonctions réactives sur la molécule hapténique; on a utilisé la méthode de fixation décrite par J.R. Little et H.N. Eisen dans Biochemistry $\underline{5}$ p3385 (1966).

Les essais ont été effectués avec des souris de souche Balb/c et la protéine-support selon l'invention était de la sérum albumine de souris (SSA), de cette même souche.

A titre de comparaison, on a aussi préparé dans les mêmes conditions des dérivés dans lesquels le TNP était fixé à de la sérum albumine bovine (BSA).

Les conjugués TNP/albumines ont été injectés aux animaux par voie intrapéritonéale, dans du sérum physiologique; les animaux témoins ont reçu des injections du même volume de sérum physiologique (PBS). Le protocole d'administration a été celui indiqué dans le Tableau 1.

TABLEAU 1

Protocole d'administration

| Injection No | Nombre de jours après la naissance | Volume de diluant (µl) | Poids de conjugué TNP/albumine (µg) |
|---|---|---|---|
| 1 | 0 | 50 | 20 |
| 2 | 2 | 50 | 20 |
| 3 | 4 | 50 | 20 |
| 4 | 6 | 50 | 20 |
| 5 | 8 | 50 | 50 |
| 6 | 10 | 50 | 50 |
| 7 | 12 | 100 | 100 |
| 8 | 14 | 100 | 100 |
| 9 | 16 | 100 | 100 |
| 10 | 18 | 100 | 100 |
| 11 | 20 | 100 | 100 |
| 12 | 22 | 100 | 100 |
| 13 | 67 | 100 | 100 |
| 14 | 90 | 100 | 100 |

Des prélèvements sanguins ont été effectués les 30ème, 60ème, 85ème et 98ème jours après la naissance. La souris est adulte au 60éme jour.

Le dosage des anticorps anti-TNP présent dans les sérums prélevés a été effectué par une méthode immunoenzymatique, connue sous le nom d'ELISA et décrite notamment dans l'article de B. GUILBERT, G. DIGHIERO et S. AVRAMEAS dans The Journal of immunology 128 (6) p 2779 - (1982).

On a opéré comme suit: des plaques de polystyrène à micropuits ont été revétues avec différents composés antigéniques en faisant incuber 1h à 37°C et une nuit à 4°C une solution de 1 µg du composé dans 1 ml de tampon carbonate/bicarbonate (0,1M ; pH = 9,5) avant d'éliminer l'excès des des réactifs par des lavages au tampon phosphate salin. Quatre types de revêtements ont été utilisés :

1) Conjugué TNP/sérum albumine de souris (SSA)

2) Conjugué TNP/sérum albumine bovine - (BSA)

3) Conjugué TNP/β-galactosidase;

4) Conjugué TNP/ovalbumine.

Les conjugués 1 et 2 sont identiques à ceux administrés aux souris pour étudier la secrétion des anticorps dirigés contre le TNP, selon le protocole du tableau 1. Ils peuvent être préparés comme suit:

a) Conjugués TNP/albumines

On dissout d'une part 20 mg d'albumine dans 1 ml de tampon carbonate/bicarbonate( 0,1M ; pH = 9,5) et d'autre part 2 mg d'acide 2,4,6-trinitrobenzène sulfonique (TNBS) dans 1 ml du même tampon. On mêlange ensuite 1 ml de chacune de ces solutions et après les avoir laissé reposer 2 heures à température ambiante, à l'abri de la lumière, on dialyse la solution pendant 48 heures, à 4°C, contre une solution de NaCl (0,15M; pH = 7,4) en changeant 2 fois de dialyse.

On obtient dans ces conditions dans le cas de la sérum albumine murine de 28 à 35 groupements trinitrophényl fixés par molécule d'albumine; dans le cas de la sérum albumine bovine de 24 à 28 groupements fixés et dans le cas de l'ovalbumine environ 12 groupements TNP fixés par moléle.

b) Conjugué TNP/$\beta$-galactosidase.

On dialyse durant 24 heures à 4°C contre une solution de NaCl( 0,15 M), une solution commerciale de $\beta$-galactosidase, puis durant 24 heures contre une solution tamponnée au borate (0,1 M; pH = 9,2). 4mg de solution de $\beta$-galactosidase, ainsi dialysée, dans 1 ml du tampon au borate sont mélangés avec 2 mg de TNBS préalablement dissous dans 1 ml du même tampon; la solution est abandonnée 30 minutes à la température du laboratoire puis dialysée pendant 48 heures à 4°C, contre une solution de NaCl ou de PBS (0,15 M; pH = 7,4). Dans ces conditions, on obtient un conjugué qui comporte environ 100 groupements de TNP par molécule de $\beta$-galactosidase.

On introduit alors dans chacun des puits ainsi revêtus 100 $\mu$l de différentes dilution du sérum à étudier (au 1/100, 1/300, 1/900 et 1/2700) dans du tampon phosphate salin contenant 0,5% de gélatine et 0,1% de Tween®20 et laisse incuber 1 heure à 37°C, avant de laver et d'introduire dans les micropuits 0,1 $\mu$g d'anticorps de mouton, dirigés contre les immunoglobulines de souris, et marqués de façon connue avec de la $\beta$-galactosidase (ou de la peroxydase si la plaque a été revêtue du conjugué TNB/$\beta$-galactosidase).

Après une heure d'incubation à 37°C, on lave les plaques abondamment pour éliminer les anticorps de mouton marqués, non fixés et on introduit le substrat de l'enzyme habituellement utilisé pour ce type de révélation puis on mesure la densité optique de la solution qui est fonction de la quantité d'enzyme fixée, qui dépend elle-même de la quantité d'anticorps anti-TNP présente dans le sérum à étudier.

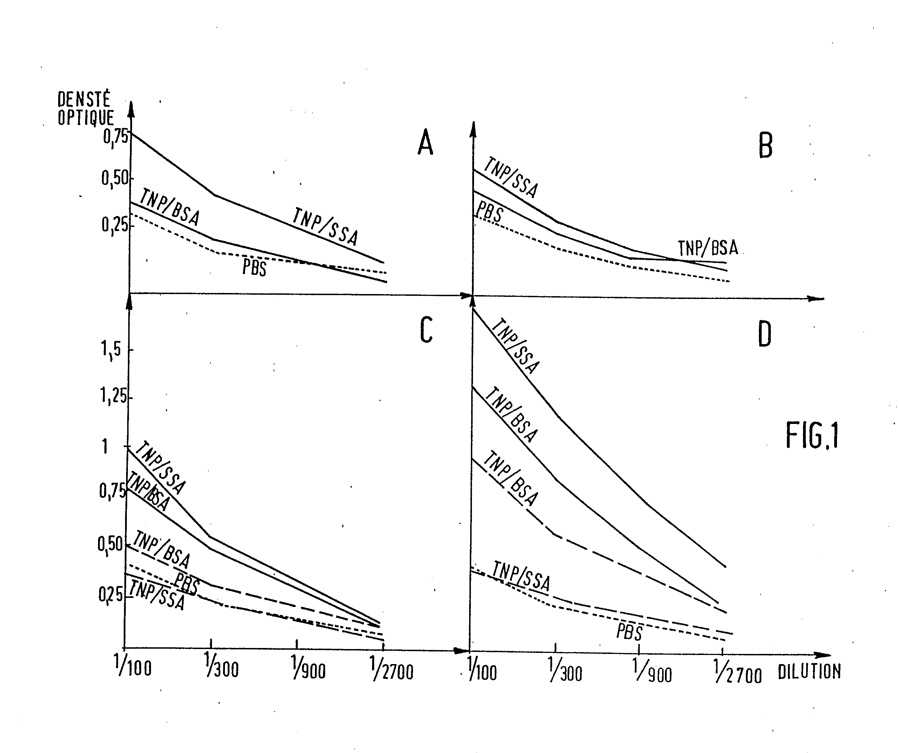

Les résultats des mesures effectuées en utilisant une plaque revêtue du conjugué TNP/$\beta$-galactosidase sont l'objet de la Figure 1, sur laquelle on a porté dans les 4 quadrants A, B, C, D en abscisse les dilutions de sérum et en ordonnée la densité optique.

Dans le quadrant A figurent les résultats obtenus lors du premier prélèvement, à 30 jours; dans le B, les résultats du 2ème prélèvement, dans le C, ceux du 3ème et en D ceux du dernier prélèvement; la courbe TNP/SSA est celle obtenue pour les animaux auxquels on a injecté le conjugué TNP/SSA suivant le protocole mentionné précédemment, alors que la courbe TNP/BSA correspond à l'administration du conjugué TNP/BSA et la courbe PBS aux témoins; les courbes en pointillé correspondent à des animaux n'ayant reçu que les injections de l'âge adulte (après le 60ème jour).

On déduit de cette figure :

-que les animaux jeunes auxquels on a injecté le conjugué TNP/SSA de l'invention présentent quelques jours après la dernière injection un taux sanguin d'anticorps circulants anti-TNP non négligeable, alors que le taux chez les animaux qui ont reçu le conjugué connu TNP/BSA est très comparable à celui des animaux témoins que n'ont reçu que le solvant de dilution

-que le taux des anticorps circulants diminue avec le temps (quadrant B par rapport à A) mais peut être sensiblement réaugmenté par des injections de rappel pratiquées à l'âge adulte (quadrants C et D) alors que les animaux contrôle, recevant leur première injection à l'âge adulte de conjugué TNP/BSA ou TNP/SSA (courbes en pointillé), ont des taux d'anticorps anti-TNP beaucoup plus faibles.

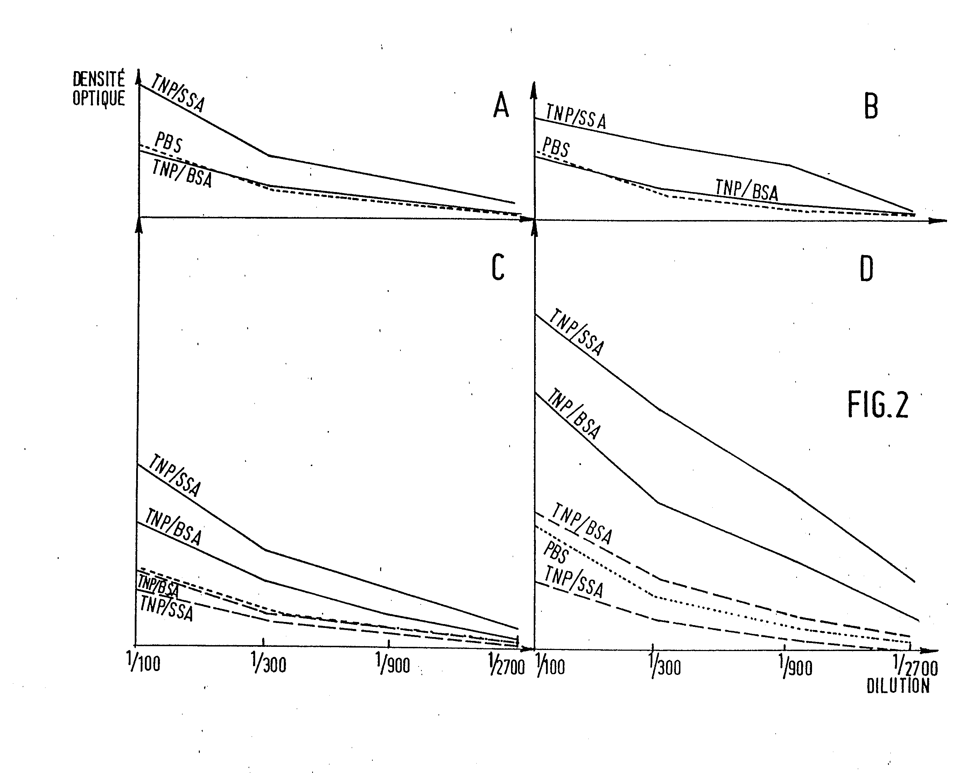

Sur la Figure 2 ont été représentés les résultats obtenus en effectuant le dosage sur une microplaque revêtue d'un conjugué TNP/ovalbumine; le fait que les figures 1 et 2 aient des aspects tout à fait comparables confirme que les anticorps qui se fixent sur les plaques sont essentiellement dirigés contre le TNP et non contre les protéines supports, que ce soit la $\beta$-galactosidase ou l'ovalbumine, puisque celles-ci ont des caractéristiques tout à fait différentes.

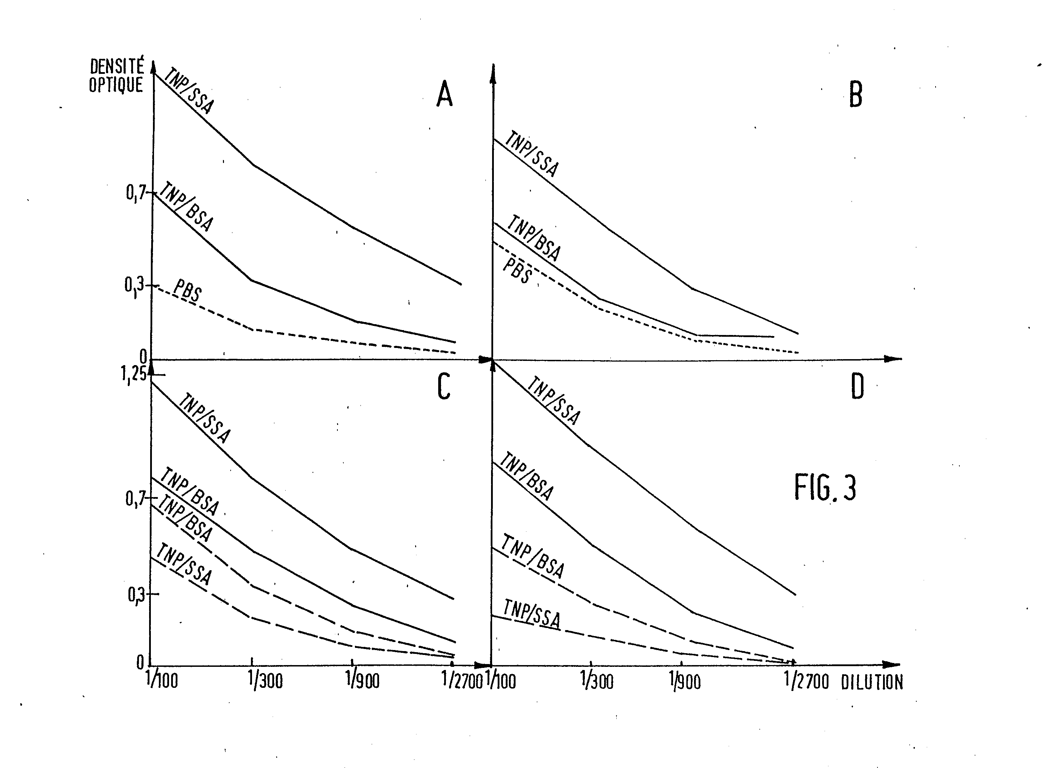

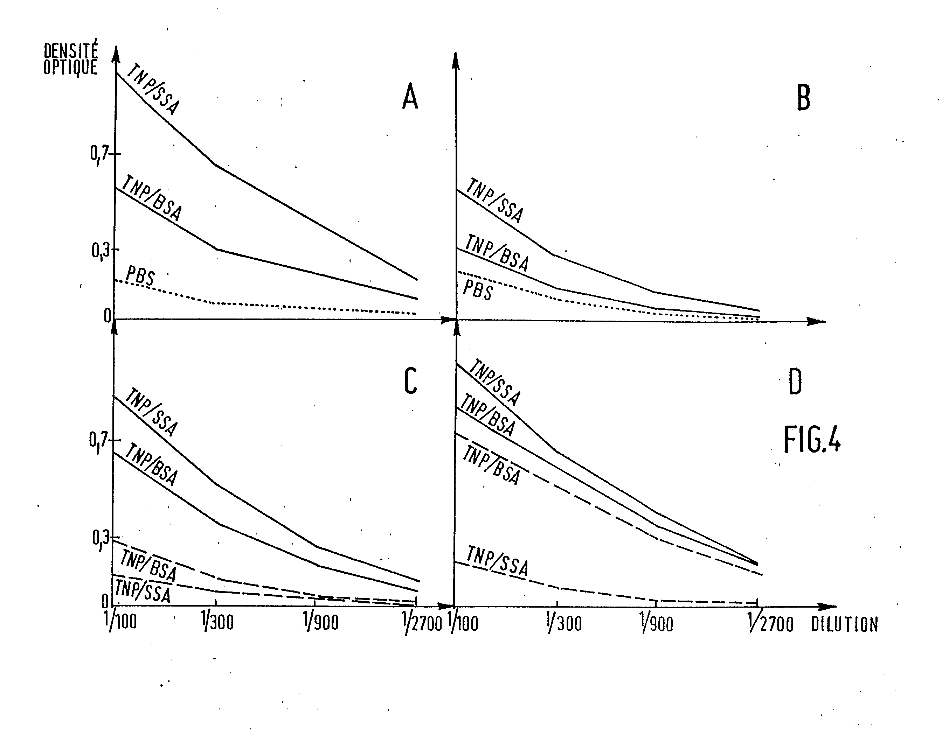

Sur la Figure 3 ont été représentés les résultats obtenus en effectuant le dosage avec une microplaque revêtue d'un conjugué TNP/SSA et sur la Figure 4 d'un conjugué TNP/BSA. Dans le cas de la Figure 3, les anticorps circulants fixés sont ceux dirigés contre le TNP, et le conjugués TNP/SSA; dans le cas de la Figure 4, ils sont dirigés contre le TNP, la BSA et leur conjugué. L'étude de ces figures permet de conclure :

-que les anticorps anti-TNP apparaissent chez l'individu jeune en quantité plus faible lorsqu'ils ont une protéine hétérologue (BSA) au lieu d'une autologue (SSA) comme protéine-support. (Figures 3 et 4, quadrant A)

-que les anticorps circulants n'ont pas disparu lorsque l'animal atteint l'âge adulte (quadrants B)

-qu'une injection de rappel à l'âge adulte déclenche une nouvelle production d'anticorps même si l'haptène est fixé sur une protéine autologue (comparaison des courbes TNP/SSA en traits pleins et en pointillés des quadrants D)

-que deux injections à l'âge adulte, sans injection préalable au nouveau-né, d'un conjugué avec une protéine autologue (TNP/SSA) ne déclenche pas de formation d'anticorps anti-TNP (courbes TNP/SSA en pointillés, quadrants D), alors que le conjugué TNP/BSA la déclenche, comme il est connu.

On opère de manière analogue avec d'autres haptènes; lorsque ceux-ci ne portent pas initialement de fonction susceptible de réagir sur la protéine, on effectuera le couplage chimique à l'aide d'une molécule réactive adaptée, par une méthode connue de l'homme de métier.

Lorsque les composés seront utilisés pour des vaccinations, on effectuera de préférence plusieurs injections à l'individu à vacciner, la première de composé se situant toujours dans la période néonatale. Lorsque les composés seront utilisés pour permettre la préparation d'anticorps particulièrement spécifiques chez l'animal, on répétera de préférence les injections pour obtenir un taux suffisant d'anticorps circulants, la première injection étant obligatoirement effectuée dans la période proche de la naissance des animaux.

**Revendications**

1. Composés pour induire chez un individu la formation d'anticorps, dirigés contre un agent antigénique, ces composés résultant de la fixation de cet agent antigénique à une molécule-support et étant caractérisés en ce que ladite molécule, administrée seule, ne déclenche pas de réaction immunitaire chez l'individu adulte auquel seront administrés les composés.

2. Composés selon la revendication 1, caractérisés en ce que la molécule-support provient d'un individu de la même espèce animale ou de l'homme dans le cas de l'induction chez l'homme.

3. Composés selon la revendication 1, caractérisés en ce que la molécule-support est une protéine.

4. Composés selon la revendication 3, caractérisés en ce que la molécule-support est la sérum albumine.

5. Composés selon l'une quelconque des revendications précédentes caractérisés en ce que l'agent antigénique est un haptène choisi parmi les peptides, les polysaccharides, les hormones et autres.

6. Composés selon l'une des revendications 1 à 4, caractérisés en ce que l'agent antigénique est un antigène figuré, tel que microorganismes, bactéries ou virus, ou un antigène soluble, tel que polysaccharides, glycolipides, glycoprotéines et protéines.

7. Composés selon l'une quelconque des revendications précédentes dans lesquels la molécule-support est couplée à l'agent antigénique par l'intermédiaire d'une molécule réactive.

8. Composition pharmaceutique pour induire une résistance à une infection chez un individu caractérisée en ce qu'elle comporte, associée à un véhicule inerte, un composé selon l'une quelconque des revendications précédentes dans lequel l'agent antigénique comporte au moins un épitope de l'agent à l'origine de l'infection.

9. Composition pharmaceutique selon la revendition 8, caractérisée en ce que la dose unitaire convient à l'immunisation d'un nouveau-né.

10. Composition pharmaceutique selon la revendication 8, caractérisée en ce que la dose unitaire convient pour effectuer une restimulation chez l'adulte.

11. Composition pour induire la formation d'anticorps de spécificité donnée chez un animal caractérisée en ce qu'elle comprend une quantité convenable d'un composé selon l'une quelconque des revendications 1 à 7, associée à un véhicule inerte.

12. Procédé pour la préparation d'anticorps in-vivo ou ex-vivo, caractérisé en ce qu'on administre au moins une fois, une composition selon la revendication 11, à des animaux nouveaux-nés, pour prélever le sérum ou les cellules immunocompétentes après achèvement de la stimulation immunitaire chez l'animal, éventuellement adulte.

FIG.1

FIG.2

FIG.3

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 302 304 (DAIICHI SEIYAKU CO., LTD.) <br> * Page 1, lignes 23-25; page 3, lignes 1-6; revendications 1,3,6 * <br><br> --- | 1-5,8, 11,12 | A 61 K 39/385 |
| A | FR-A-2 359 851 (SLOAN-KETTERING INSTITUTE FOR CANCER RESEARCH) <br> * Page 1, lignes 34-38; page 2, lignes 14-26; page 3, ligne 27 - page 4, ligne 1; revendications 1-5 * <br><br> --- | 1-12 | |
| A | FR-A-2 028 475 (YEDA RESEARCH AND DEVELOPMENT CO., LTD.) <br><br> * Page 1, lignes 1-3,16-22; revendications 1-5 * <br><br> --- | 1,3,4, 6-8,11 ,12 | |
| A | US-A-4 004 979 (S. AVRAMEAS et al.) <br> * Colonne 1, lignes 8-13; colonne 2, lignes 44-48, ligne 61 - colonne 3, ligne 9; colonne 3, ligne 62 - colonne 4, ligne 4; colonne 12, exemple 8 * <br><br> --- | 1-12 | DOMAINES TECHNIQUES RECHERCHES (Int. Cl.4) <br><br> A 61 K |
| A | FR-A-2 527 447 (J. LEGUERN) <br> * Page 1, lignes 1-3,17-22, page 2, lignes 1-4; revendication * <br><br> --- -/- | 1-11 | |

Le présent rapport de recherche a été établi pour toutes les revendications

| Lieu de la recherche | Date d'achèvement de la recherche | Examinateur |
|---|---|---|
| LA HAYE | 31-07-1986 | CHARLES D.J.P.I.G. |

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 122 132  (KUREHA KAGAKU KOGYO K.K.) <br> * Page 3, lignes 2-17; page 4, lignes 5-9; page 5, lignes 10-23; page 10, lignes 13-20; page 14, lignes 10-19; revendications 1-4 * <br><br> ----- | 1-3,5 | |

**DOCUMENTS CONSIDERES COMME PERTINENTS**   Page  2

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 31-07-1986 | CHARLES D.J.P.I.G. |